# EUROPÄISCHE PATENTSCHRIFT

(11) **EP 1 604 251 B1**
(45) Veröffentlichungstag und Bekanntmachung des Hinweises auf die Patenterteilung: **14.07.2010**
(21) Anmeldenummer: 04722274.0
(22) Anmeldetag: 22.03.2004
(51) Int. Cl.: G05B 13/02

(54) **VERFAHREN ZUM AUSLÖSEN INSASSENUNTERSTÜTZTER MASSNAHMEN IN EINEM FAHRZEUG**
METHOD FOR INITIATING OCCUPANT-ASSISTED MEASURES INSIDE A VEHICLE
PROCEDE POUR DECLENCHER DES MESURES DE PROTECTION DES OCCUPANTS DANS UN VEHICULE

(30) Priorität: 20.03.2003 DE 10312519
(43) Veröffentlichungstag der Anmeldung: 14.12.2005
(73) Patentinhaber: Fraunhofer-Gesellschaft zur Förderung der angewandten Forschung e.V., 80686 München (DE)
(72) Erfinder: MÜLLER, Klaus-Robert, 12159 Berlin (DE); BLANKERTZ, Benjamin, 10247 Berlin (DE); CURIO, Gabriel, 14167 Berlin (DE)
(74) Vertreter: von Kreisler Selting Werner
(86) Internationale Anmeldenummer: PCT/EP2004/003012
(87) Internationale Veröffentlichungsnummer: WO 2004/083972

(56) Entgegenhaltungen:
- DE-A- 19 702 748
- DE-C- 19 801 009
- US-A- 5 311 877
- US-A- 5 638 826
- US-A1- 2002 077 534
- US-B1- 6 349 231

## Beschreibung

Die Erfindung betrifft ein Verfahren zum Auslösen insassenunterstützter Maßnahmen in einem Fahrzeug.

Aus DE 198 01 009 C1 ist ein Verfahren bekannt, bei dem eine Not- oder Stresssituation des Fahrers eines Fahrzeuges erkannt und eine einen Bremsvorgang einleitende bzw. durchführende Einrichtung unterstützend betätigt wird. Die Not- bzw. Stresssituation des Fahrers wird dabei anhand von Sensoren detektiert, die eine Änderung des Blutdrucks und/oder eine Änderung des Pulses und/oder eine Änderung der Pupille und/oder eine Änderung des Gesichtsausdrucks und/oder eine Änderung des Lidreflexes und/oder eine Muskelkontraktion, vorzugsweise der Hand, und/oder eine Änderung des Hautwiderstands und/oder eine Änderung der Schweißsekretion erfassen.

Die Zeit bis zur Entstehung einer der zuvor genannten Körperreaktionen auf eine vom Fahrer empfundene Not- bzw. Stresssituation hin führt dabei zu einer verzögerten unterstützenden Einleitung bzw. Unterstützung des Bremsvorganges, was nachteilig sein kann.

Aus DE 197 02 748 A1 ist es ferner bekannt, dass der Zustand des Führers eines Fahrzeuges, z. B. eines Zuges, durch z. B. die Erfassung der Hirnströme des Führers überwacht wird.

Schließlich ist es aus US-B-6 349 231 bekannt, den Willen einer Person, die sich z. B. in einem automatischen Rollstuhl befindet, durch Analyse von Hirnstromsignalen zu ermitteln, um damit z. B. den Rollstuhl zu steuern.

Eine Aufgabe der Erfindung ist es, ein Verfahren zum Auslösen insassenunterstützender Maßnahmen in einem Fahrzeug anzugeben, bei dem die Zeit zwischen der Bildung der Intention des z. B. Fahrers des Fahrzeuges und der ein zuleitenden Maßnahme verkürzt und diese damit quasi ohne Zeitverzögerung eingeleitet werden kann.

Zur Lösung dieser Aufgabe wird mit der Erfindung ein Verfahren zum Auslösen insassenunterstützter Maßnahmen in einem Fahrzeug vorgeschlagen, bei dem
- Hirnstrom-Signale mindestens eines Fahrzeuginsassen, insbesondere des Fahrers, messtechnisch erfasst werden,
- anhand der Hirnstrom-Signale die Intention des Fahrzeuginsassen durch Echtzeitverarbeitung, ermittelt wird, wobei die Bildung motorischer Intentionen und Bewegungsvorbereitungen des Fahrzeuginsassen durch Extraktion von als Einzelereignis identifizierbaren Korrelaten aus dem Hirnstrom-Signal ermittelt wird, und
- basierend auf der auf diese Weise ermittelten motorischen Intention und Bewegungsvorbereitung des Fahrzeuginsassen Maßnahmen zum Überführen des augenblicklichen Zustandes des Fahrzeuges in einen der motorischen Intention des Fahrzeuginsassen angepassten Zustand des Fahrzeugs im voraus ausgelöst werden.

Vorteilhafte Weiterbildungen der Erfindung sind in den Unteransprüchen angegeben.

Nach der Erfindung werden die handlungsspezifischen Intentionen der Insassen bzw. des Fahrers anhand von deren/dessen Hirnströmen erkannt. Dies erfolgt somit zum frühestmöglichen Zeitpunkt, womit Zeitverzögerungen, die z. B. bis zur Entstehung von Sekundarreaktionen des Körpers gegeben sind, vermieden werden. Ferner lassen sich auch Intentionen detektieren, die keinerlei Sekundärreaktionen des Körpers hervorrufen. Z. B. kann anhand der Hirnströme erkannt werden, wie der Fahrer das Fahrzeug zu lenken beabsichtigt, um je nach Art und Weise des Lenkmanövers Fahrwerksstabilisationssysteme optimal vorzubereiten.

Mit der Erfindung wird also ein Verfahren zum Einsatz in Fahrzeugen zur Bereitstellung einer verbesserten Fahrer-Fahrzeug-Schnittstelle durch Auswertung von Hirnströmen wie z. B. EEG, MEG, NIRS, fMRI und/oder um EMG vorgeschlagen.

Das erfindungsgemäße Verfahren hat unter anderem die Eigenschaft, dass Fahrerverhalten ganz allgemein und Fahrer-Reaktionsfehler und Reaktionsverzögerungen im speziellen detektiert und analysiert werden und damit als neuartiges Multipurposefeature für eine verbesserte Fahrzeugsicherheit einem nachfolgenden Sicherheitssystem als Eingabe zur Verfügung stehen. Das Verfahren kann in einem Fahrzeug unter anderem eingesetzt werden für
1. unfallpräventive Sicherheitsmaßnahmen wie
   a) automatische Gurtstraffung
   b) Sitzoptimierung
   c) brems/lenkungsvorbereitende Optimierung der Fahrzeugreagibilität
   d) Voroptimierung der Fahrzeugdynamik bei zeitkritischen Entscheidungen
   e) alle prädiktiven Sicherheitsvorkehrungen.
2. Fahrerbasierte Verifikation maschinell erkannter Gefahrensituationen, wie z.B.
   a) Detektion eines kongruenten motorischen Intentionsaufbaus
   b) Situationsmodellierung und Validierung.
3. Kontinuierliches Vigilanzmonitoring.

Der Erfindung und ihre Grundlagen sowie Grundzüge werden nachfolgend eingehender beschrieben.

Mit der Erfindung wird eine grundsätzlich neue Qualität von Mensch-Maschine-Schnittstellen durch die Kombination hirnphysiologischer Erkenntnisse und algorithmischer Weiterentwicklungen in der Informationstechnik ermöglicht, indem das Konzept einer direkten Umsetzung von Hirnsignalen in maschinenbezogene Steuerbefehle in einen Brain-Computer Interface (BCI) als Echtzeit-Implementation realisiert wird. Als nicht-invasive und prinzipiell alltagstaugliche Messmethode wird dabei z.B. das Multi-Kanal-EEG mit einer Zeitauflösung im Millisekundenbereich verwendet. Der methodische Ansatz beruht auf robusten Algorithmen des maschinellen Lernens und der Signalverarbeitung zur Extraktion, Identifikation und Klassifikation von EEG-Hirnsignalen, die Intentionen natürlicher Bewegungen in psychophysiologisch wohldefinierten Interaktionssituationen zwischen Mensch und Umwelt abbilden. Ein weiteres charakteristisches Merkmal des hier verwendeten BBCI liegt in der Ausrichtung auf eine für den Nutzer optimierte Trainingssituation, bei der im Gegensatz zu anderen BCI-Verfahren nicht mehrere Trainingssessions des Nutzers erforderlich sind, sondern lediglich eine einzige ca. zwanzigminütige Trainingsphase als Ausgangsmaterial für den Lernalgorithmus benötigt wird (siehe Blankertz, B., Curio, G., Müller, K.-R. (2003), Classifying Single Trial EEG: Towards Brain Computer Interfacing, Advances in Neural Information Processing Systems 14, eds. T.G. Dietterich, S. Becker and Z. Ghahramani, MIT PRSS: Cambridge, MA, 157-164; Dornhege, G., Blankertz, B., Curio, G., Müller, K.-R., Combining features for BCI, Advances in Neural Information Processing Systems 15, eds. S. Becker, S. Thrun and K. Obermayer, MIT Press: Cambridge, MA. (2003)).

Für ein BCI liegen international bislang schon wohldefinierte Anwendungsperspektiven im klinischen Einsatz für gelähmte Patienten vor, insbesondere z.B. bei kompletten Querschnittslähmungen. Mit der Erfindung wird erstmals die Möglichkeit aufgezeichnet, bei zeitkritischen Echtzeit-Applikationen, wie sie typischerweise z.B. bei Fahrer-Fahrzeug-Schnittstellen gegeben sind, neuartige Verfahrensansätze zu realisieren:
1. In der psychophysiologischen Forschung zur Aufklärung und Anwendung von Fahrer-Reaktionsfehlern und -Reaktionsverzögerungen können nun erstmals, sowohl in virtuellen Fahrsimulationen wie auch in realen Fahrsituationen, die motorischen Reaktionsintentionen des Fahrers mit hoher Zeitauflösung im Millisekundenbereich als *ungemittelte Einzelereignisse* erfasst und auf diese Weise *in Abhängigkeit vom aktuell variierenden perzeptuellen Kontext* (multimodale Umgebungsinformationen sowie Instrumentensignale) analysiert werden.
2. Im Einsatz als Fahrerassistenzsystem können Konzepte der "Integrierten Sicherheit" um neuartige Komponenten für eine kontinuierlich ('on-thefly') fortlaufende Fahrer-Modellierung erweitert werden:
   a) Die als Einzelereignis identifizierbaren EEG-Korrelate von Intentionsbildung und spezifischen Bewegungsvorbereitungen können aufgrund der BBCI-Echtzeitfähigkeit als *neuartige Eingangsgröße* dienen *für Konzepte der unfallpräventiven Sicherheit,* bei Automobilen beispielsweise motorische Gurtstraffung, Sitzoptimierung oder Brems-/Lenkungs-vorbereitende Optimierung der Fahrzeugreagibilität.
   b) Darüber hinaus kann eine *schnellstmögliche Fahrer-basierte 'Verifikation' einer maschinellen (z.B. visuellen) Gefahrenerkennung* durch Detektion eines kongruenten motorischen Intentionsaufbaus des Fahrers erfolgen und eine dementsprechend validierte Situationsmodellierung ermöglicht.
   c) Insbesondere können *zeitkritische Entscheidungsalternativen,* wie z.B. eine situativ zwingende Auswahl zwischen Notfallbremsung und gerichtetem Ausweichmanöver, die rechtlich dem Fahrer vorzubehalten sind, schon Zehntelsekunden vor der eigentlichen Reaktionsbewegung des Fahrers prognostiziert werden, indem die entsprechenden motorischen Intentionen aus dem EEG-Signal des Fahrers extrahiert und für Zwecke einer Voroptimierung der Fahrzeugdynamik genutzt werden.

Als additiver Vorteil dieses EEG-basierte BCI-Ansatzes ist das weitergreifende *multi-purpose feature* zu nennen, dass aus den EEG-Daten neben den hier definierten neuartigen Applikationen schon früher etablierte Konzepte zum kontinuierlichen Fahrer-Vigilanzmonitoring nahtlos integriert werden können.

## Patentansprüche

1. Verfahren zum Auslösen insassenunterstützter Maßnahmen in einem Kraftfahrzeug, bei dem
- Hirnstrom-Signale mindestens eines Fahrzeuginsassen, insbesondere des Fahrers, messtechnisch erfasst werden,
- anhand der Hirnstrom-Signale die Intention des Fahrzeuginsassen durch Echtzeitverarbeitung ermittelt wird, wobei die Bildung motorischer Intentionen und Bewegungsvorbereitungen des Fahrzeuginsassen durch Extraktion von als Einzelereignis identifizierbaren Korrelaten aus dem Hirnstrom-Signal ermittelt wird, und
- basierend auf der auf diese Weise ermittelten motorischen Intention und Bewegungsvorbereitung des Fahrzeuginsassen Maßnahmen zum Überführen des augenblicklichen Zustandes des Kraftfahrzeuges in einen der motorischen Intention des Fahrzeuginsassen angepassten Zustand des Kraftfahrzeugs im voraus ausgelöst werden.

2. Verfahren nach Anspruch 1, **dadurch gekennzeichnet, dass** die physiologischen Signale nichtinvasiv ermittelt werden.

3. Verfahren nach Anspruch 1 oder 2, **dadurch gekennzeichnet, dass** es sich bei den Hirnstrom-Signalen um Hirnsignale wie z. B. EEG, MEG, NIRS, fMRI und/oder um EMG handelt.

4. Verfahren nach einem der Ansprüche 1 bis 3, **dadurch gekennzeichnet, dass** die Echtzeitverarbeitung der Messsignale durch Methoden der Signalverarbeitung und/oder des maschinellen Lernens erfolgt, die es ermöglichen, die Messsignale als Einzelsignale und ohne langwieriges Training des Fahrzeuginsassen auszuwerten.

5. Verfahren nach Anspruch 4, **dadurch gekennzeichnet, dass** die Methoden der Signalverarbeitung zur adaptiven Merkmalsextraktion aus den Messsignalen mindestens eines der nachfolgenden Merkmale aufweist:
a) Filterung (räumlich und im Frequenzbereich) und Downsampling,
b) Zerlegung bzw. Projektion,
c) Bestimmung von räumlichen, zeitlichen oder raum-zeitlichen Komplexitätsmaßen,
d) Bestimmung von Kohärenzmaßen (bezogen auf Phase oder Band-Energie) zwischen Eingangssignalen.

6. Verfahren nach Anspruch 5, **dadurch gekennzeichnet, dass** die Filterung mindestens eines der nachfolgenden Merkmale aufweist:
a) Wavelet und Fourierfilter (short-time),
b) FIR und IIR Filter,
c) Laplace und Common Avarage Reference Filter,
d) Glättungsverfahren.

7. Verfahren nach Anspruch 5, **dadurch gekennzeichnet, dass** die Zerlegung bzw. Projektion mindestens eines der nachfolgenden Merkmale aufweist:
a) Independent Component Analyse und Hauptkomponentenanalyse,
b) Projection Pursuit Technik,
c) Sparse Decomposition Techniken,
d) Common Spatial Patterns Techniken,
e) Common Subspace Decomposition Techniken,
f) (Bayessche) sub-space regularization Techniken.

8. Verfahren nach Anspruch 4 oder einem der vorhergehenden Ansprüche, soweit auf Anspruch 4 rückbezogen, **dadurch gekennzeichnet, dass** die Methode des maschinellen Lernens eine Klassifikation und/oder Regression umfasst, und zwar unter Einsatz von
a) kernbasierten linearen und nichtlinearen Lernmaschinen (z.B. Support Vector Maschinen, Kern Fisher, Linear Programming Machines),
b) Diskriminanzanalysen,
c) neuronalen Netzen,
d) Entscheidungsbäumen,
e) allgemein allen linearen und nicht linearen Klassifikationsmethoden auf die durch Signalvorverarbeitung gewonnenen Merkmale.

9. Verfahren nach einem der Ansprüche 1 bis 8, **dadurch gekennzeichnet, dass** es sich bei den auslösenden Maßnahmen um unfallpräventive Sicherheitsmaßnahmen wie
a) automatische Gurtstraffung,
b) Sitzoptimierung,
c) brems/lenkungsvorbereitende Optimierung der Fahrzeugreagibilität,
d) Stabilitätsvorberechnungen,
e) Voroptimierung der Fahrzeugdynamik bei zeitkritischen Entscheidungen,
f) alle prädiktiven Sicherheitsvorkehrungen handelt.

10. Verfahren nach einem der Ansprüche 1 bis 9, **dadurch gekennzeichnet, dass** die anhand der Hirnstrom-Signale ermittelte bzw. abgeschätzte Intention der Verifikation maschinell erkannter Gefahrensituationen dient, und zwar insbesondere durch Detektion eines kongruenten motorischen Intentionsaufbaus und Situationsmodellierung und Validierung.

11. Verfahren nach einem der Ansprüche 1 bis 10, **gekennzeichnet durch** den Einsatz und die Integration in ein kontinuierliches Vigilanzmonitoring.

12. Verfahren nach einem der Ansprüche 1 bis 11, **dadurch gekennzeichnet, dass** die auszulösenden Maßnahmen anhand einer Mittlung der Intentionen mehrerer Fahrzeuginsassen ergriffen werden.

## Claims

1. A method for initiating occupant-assisted measures inside a motor vehicle, wherein
- cerebral-current signals of at least one vehicle occupant, particularly of the driver, are detected by a measurement technique,
- on the basis of the cerebral-current signals, the intention of the vehicle occupant is estimated or detected by real-time processing, the forming of motoric intentions and the preparation of movements by the vehicle occupant being determined by an extraction of correlates from the cerebral-current signals, which correlates can be identified as an individual event, and
- on the basis of the determined motoric intention and preparation of movement by the vehicle occupant, measures for transferring the current state of the vehicle into a state of the vehicle matched to the motoric intention of the vehicle occupant are initiated in advance.

2. The method according to claim 1, **characterized in that** the physiological signals are detected non-invasively.

3. The method according to claim 1 or 2, **characterized in that** the cerebral-current signals are cerebral signals such as e.g. EEG, MEG, NIRS, fMRI and/or EMG.

4. The method according to any one of claims 1 to 3, **characterized in that** the real-time processing of the measurement signals is performed by use of methods of signal processing and/or machine learning which allow an evaluation of the measurement signals as individual signals and without extensive training of the occupant of the vehicle.

5. The method according to claim 4, **characterized in that** the methods for signal processing for adaptive feature extraction from the measurement signals comprise at least one of the following features:
a) filtration (spatial and in the frequency range) and downsampling,
b) splitting and projection, respectively,
c) determination of spatial, temporal or spatio-temporal complexity dimensions,
d) determination of coherence dimensions (related to phase or band energy) between input signals.

6. The method according to claim 5, **characterized in that** the filtration comprises at least one of the following features:
a) wavelet or Fourier filter (short-time),
b) FIR or IIR filter,
c) Laplace and common average reference filter,
d) smoothing method.

7. The method according to claim 5, **characterized in that** the splitting and projection, respectively, comprises at least one of the following features:
a) independent component analysis and main component analysis,
b) projection pursuit technique,
c) sparse decomposition techniques,
d) common spatial patterns techniques,
e) common sub-space decomposition techniques,
f) (Bayes') sub-space regularization techniques.

8. The method according to claim 4 or any one of the preceding claims as far as dependent on claim 4, **characterized in that** the machine learning method comprises a classification and/or regression, notably by use of
a) core-based linear and non-linear learning machines (e.g. support vector machines, Kern Fisher, linear programming machines),
b) discriminance analyses,
c) neuronal networks,
d) decision trees,
e) generally, all linear and non-linear classification methods for the features obtained by signal processing.

9. The method according to any one of claims 1 to 8, **characterized in that** the initiating measures are accident-preventive measures such as e.g.
a) automatic safety belt tightening,
b) seat optimization,
c) optimization of the vehicle reagibility to prepare a braking/steering operation,
d) stability computations,
e) pre-optimization of the vehicle dynamics in case of time-critical decisions,
f) all predicative safety measures.

10. The method according to any one of claims 1 to 9, **characterized in that** the intention detected or estimated on the basis of the cerebral-current signals serves for the verification of device-detected hazard situations, particularly by detection of a congruent motor intention build-up and situation modeling and validating.

11. The method according to any one of claims 1 to 10, **characterized by** use and integration in continuous vigilance monitoring.

12. The method according to any one of claims 1 to 11, **characterized in that** the measures to be initiated are taken on the basis of an averaging of the intentions of a plurality of vehicle occupants.

## Revendications

1. Procédé pour déclencher des mesures assistées par les occupants dans un véhicule motorisé, dans lequel
- des signaux de courant cérébral d'au moins un occupant du véhicule, notamment du conducteur, sont détectés par des techniques de mesurage,
- l'intention de l'occupant du véhicule est déterminé à partir desdits signaux de courant cérébral par traitement de données en temps réelle, la formation des intentions motrices et les préparations de mouvements de l'occupant du véhicule étant déterminées, à partir des signaux de courant cérébrale, par extraction de corrélats identifiables come évènements singulières, et
- à partir de ladite intention motrice et de la préparation de mouvements de l'occupant du véhicule ainsi déterminées, des mesures sont déclenchées par avance pour transférer l'état actuel du véhicule dans un état du véhicule adapté à l'intention motrice de l'occupant du véhicule.

2. Procédé selon la revendication 1, **caractérisé en ce que** les signaux physiologiques sont déterminés d'une manière non-invasive.

3. Procédé selon la revendication 1 ou 2, **caractérisé en ce que** les signaux de courant cérébral son des signaux cérébraux comme, par exemple, EEG, MEG, NIRS, fMRI et/ou EMG.

4. Procédé selon l'une des revendications 1 à 3, **caractérisé en ce que** le traitement en temps réel des signaux de mesurage est réalisé par des méthodes de traitement de signaux et/ou d'apprentissage symbolique automatique, permettant l'évaluation des signaux de mesurage comme signaux individuels et sans un long entrainement de l'occupant du véhicule.

5. Procédé selon la revendication 4, **caractérisé en ce que** les méthodes de traitement de signaux pour l'extraction adaptive de caractéristiques à partir des signaux de mesurage comprennent au moins une des caractéristiques suivantes:
a) filtration (spatial et dans la gamme de fréquence) et downsampling,
b) décomposition ou projection, respectivement,
c) détermination de mesures de complexité spatiales, temporelles ou spatio-temporelles,
d) détermination de mesures de cohérence (rélatif à la phase ou énergie de bande) entre des signaux d'entrée.

6. Procédé selon la revendication 5, **caractérisé en ce que** la filtration comprend au moins une des caractéristiques suivantes:
a) filtre d'ondelette et filtre de Fourier (court terme)
b) filtre FIR et IIR,
c) filtre de Laplace et filtre du type "Common Average Reference",
d) méthode de lissage.

7. Procédé selon la revendication 5, **caractérisé en ce que** la décomposition ou la projection respectivement comprend au moins une des caractéristiques suivantes:
a) analyse en composantes indépendantes et analyse en composantes principales,
b) technique de poursuite de projection,
c) techniques du type "Sparse Decomposition",
d) techniques du type "Commom Spatial Patterns",
e) techniques du type "Common Subspace Decomposition",
f) techniques de "Subspace Regularization (Bayes)".

8. Procédé selon la revendication 4 ou une des revendications précédentes comme référée à la revendication 4, **caractérisé en ce que** la méthode d'apprentissage symbolique automatique comprend une classification et/ou une régression, utilisant:
a) machines d'apprentissage linéaires et non-linéaires, basées sur des noyaux (p. ex, machines support vecteur, Noyau-Fisher, machines à programmation linéaire),
b) analyses de discriminance,
c) réseaux neuronales,
d) arbres de décision,
e) généralement, toutes les méthodes de classification linéaires et non-linéaires appliqués sur les caractéristiques obtenues par traitement de signaux.

9. Procédé selon l'une quelconque des revendications 1 à 8, **caractérisé en ce que** les mesures provoquant le déclenchement sont des mesures de sécurité destinées à la prévention d'accidents, comme
a) prétension automatique de ceinture,
b) optimisation de siège,
c) optimisation de la capacité de réaction du véhicule en préparation de freinage et de direction,
d) calculs préalables de stabilité,
e) optimisation préalable de la dynamique du véhicule en cas de décisions urgents,
f) toutes les préventions de sécurité prédictives.

10. Procédé selon l'une quelconque des revendications 1 à 9, **caractérisé en ce que** l'intention déterminée ou estimée a partir des signaux de courant cérébral sert à vérifier des situations dangereuses détectées mécaniquement, notamment par détection d'une initialisation congruente d'une intention motrice et par modelage de situation et par validation.

11. Procédé selon l'une quelconque des revendications 1 à 10, **caractérisé par** l'utilisation d'un monitoring de vigilance continu et l'intégration dans ledit monitoring.

12. Procédé selon l'une quelconque des revendications 1 à 11, **caractérisé en ce que** les mesures à déclencher sont réalisées basé sur la moyenne des intentions de plusieurs occupants du véhicule.
